# EUROPÄISCHE PATENTANMELDUNG

(11) **EP 4 475 138 A1**
(43) Veröffentlichungstag der Anmeldung: **11.12.2024**
(21) Anmeldenummer: 23177987.7
(22) Anmeldetag: 07.06.2023
(51) Int. Cl.: G16H 40/40, G16H 40/63

(54) **MEDIZINISCHES GERÄT MIT WORKSTATION-FUNKTION IN EINEM DATENVERARBEITUNGSSYSTEM**

(71) Anmelder: B. Braun Melsungen AG, 34212 Melsungen (DE)
(72) Erfinder: STEUBE, Johannes, 34593 Knüllwald (DE); BUERGER, Maria, 34323 Malsfeld (DE); SCHMOLL, Horst, 34302 Guxhagen (DE); PAETZOLD, Matthias, 34212 Melsungen (DE); ANGERSBACH, Klaus, 34326 Morschen (DE); LOLL, Stephen, 36199 Rotenburg an der Fulda (DE)
(74) Vertreter: Winter, Brandl - Partnerschaft mbB

(57) **Zusammenfassung**

Die vorliegende Offenbarung betrifft ein medizinisches System (1) aus einem medizinischen Gerät (2), insbesondere einer Spritzen- oder Infusionspumpe, mit einer Bedieneinheit / Interaktionseinheit (6), und einer Datenverarbeitungseinheit (4), insbesondere einem (Cloud-)Server und/oder einer digitalen Plattform, mit einer Ein- und/oder Ausgabeeinheit (8), in die individualisierte Informationen eingebbar sind, wobei das medizinische Gerät (2) anhand der individualisierten Informationen von der Datenverarbeitungseinheit (4) über eine Datenverbindung (10) ansteuerbar ist und eingegebene und übermittelte Informationen an der Bedieneinheit (6) anzeigbar sind, und wobei die Datenverarbeitungseinheit (4) über individualisierte Informationen, die in die Bedieneinheit (6) eingebbar und über die Datenverbindung (10) an die Datenverarbeitungseinheit (4) übermittelbar sind, ansteuerbar ist und die übermittelten individualisierten Informationen eine Aktion an der Datenverarbeitungseinheit (4) bewirken.

## Beschreibung

### Technisches Gebiet

Die vorliegende Offenbarung betrifft ein System aus einem medizinischen (Behandlungs-)Gerät, insbesondere einer Spritzen- oder Infusionspumpe, einer Datenverarbeitungseinheit und einer Datenverbindung. Daneben betrifft die vorliegende Offenbarung ein (medizinisches) Kennzeichnungsverfahren, ein (medizinisches) Meldeverfahren, ein computerlesbares Speichermedium sowie ein Computerprogramm gemäß den Oberbegriffen der nebengeordneten Ansprüche.

### Technischer Hintergrund der Offenbarung

Bekannte medizinische Geräte, wie beispielsweise medizinische Pumpen, insbesondere Spritzen- oder Infusionspumpen, können über eine Datenverbindung mit einer (zentralen) Datenverarbeitungseinheit bzw. einer (zentralen) IT-Plattform verbunden sein. Dabei können mehrere medizinische Geräte mit einer einzigen (zentralen) Datenverarbeitungseinheit verbunden sein. Die Datenverarbeitungseinheit kann auch ein (zentraler) (Cloud-)Server mit entsprechender Hardware für Ein- und/oder Ausgaben sein. Über die Datenverbindung können beispielsweise Software-Updates von der Datenverarbeitungseinheit auf das jeweilige medizinische Gerät aufgespielt oder eine (Software-)Lizenz auf dem jeweiligen Gerät freigeschaltet werden. Auf der anderen Seite können die medizinischen Geräte Therapiedaten, Alarme oder Warnungen an die Datenverarbeitungseinheit übermitteln, die als Meldung an einer Ein- oder Ausgabeeinheit der Datenverarbeitungseinheit, beispielsweise an einem Bildschirm, angezeigt werden. Somit können die Alarme oder Warnungen der einzelnen medizinischen Geräte an einen Nutzer ausgegeben werden.

Bei den bekannten Geräten ist das medizinische Gerät an sich also passiv. D. h., das medizinische Gerät kann Daten sammeln und an die Datenverarbeitungseinheit übermitteln. Ferner kann das medizinische Gerät mit der Datenverarbeitungseinheit kommunizieren und entsprechende Ausgaben an den Nutzer ausgegeben. Eine aktive Bedienung oder Beeinflussung der Datenverarbeitungseinheit über das medizinische Gerät (etwa im Sinne einer Workstation) ist aber nicht möglich.

### Zusammenfassung der Offenbarung

Es ist daher die Aufgabe der vorliegenden Offenbarung, die Nachteile aus dem Stand der Technik zu überwinden oder zumindest zu vermindern und insbesondere ein medizinisches System, ein (medizinisches) Kennzeichnungsverfahren, ein (medizinisches) Meldeverfahren, ein computerlesbares Speichermedium sowie ein Computerprogramm bereitzustellen, welches eine aktive Steuerung einer (zentralen) Datenverarbeitungseinheit insbesondere an einem medizinischen Gerät der vorstehend genannten Gattung (als Workstation) ermöglicht.

Diese Aufgaben der vorliegenden Offenbarung werden hinsichtlich eines medizinischen Systems offenbarungsgemäß durch die Merkmale des Anspruchs 1 gelöst, hinsichtlich eines (medizinischen) Kennzeichnungsverfahrens offenbarungsgemäß durch die Merkmale des Anspruchs 10 gelöst, hinsichtlich eines (medizinischen) Meldeverfahrens offenbarungsgemäß durch die Merkmale des Anspruchs 12, hinsichtlich eines computerlesbaren Speichermediums offenbarungsgemäß durch die Merkmale des Anspruchs 14 und hinsichtlich eines Computerprogramms offenbarungsgemäß durch die Merkmale des Anspruchs 15 gelöst.

Die vorliegende Offenbarung betrifft ein medizinisches System zumindest aus einem medizinischen Gerät, insbesondere einer Spritzen- oder Infusionspumpe, mit einer Bedieneinheit / Interaktionseinheit, einer (zentralen) Datenverarbeitungseinheit, insbesondere einen (Cloud-)Server bzw. eine digitale Plattform, und einer Datenverbindung, die das medizinische Gerät und die Datenverarbeitungseinheit verbindet. Die Datenverarbeitungseinheit hat eine Ein- und ggf. Ausgabeeinheit, in die (Geräte-) individualisierte Informationen zumindest eingebbar sind. Die (Geräte-) individualisierten Informationen sind über die Datenverbindung von der Datenverarbeitungseinheit an das zumindest eine (ausgewählte) medizinische Gerät übermittelbar und das medizinische Gerät ist anhand der eingegebenen und übermittelten Informationen von der Datenverarbeitungseinheit (individuell) ansteuerbar. Die Bedieneinheit des medizinischen Geräts ist ferner dafür vorbereitet, die an der Datenverarbeitungseinheit eingegebenen und übermittelten Informationen anzuzeigen. Des Weiteren ist das medizinische System dafür vorbereitet, individualisierte (Steuerungs-) Informationen, die in die Bedieneinheit (unmittelbar/direkt oder über ein transportables, ggf. kabellos verbundenes Eingabegerät wie Tablet, Laptop etc.) eingebbar sind bzw. eingegeben werden, über die Datenverbindung an die Datenverarbeitungseinheit zu übermitteln, wobei die Eingabe der individualisierten Informationen in die Bedieneinheit eine (beeinflussende) Aktion an der Datenverarbeitungseinheit bewirkt (d.h. die Datenverarbeitungseinheit beispielsweise derart umprogrammiert wird, dass diese ggf. eine geänderte Steuerung zumindest dieses einen individuellen Geräts fortan durchführt).

Demzufolge ist unter einer Geräte-individualisierten Information beispielsweise eine Gerätekennung, ein Steuerungsbefehl oder ein Steuerungsparameter oder dergleichen zu verstehen, der ausschließlich dieses eine medizinische Gerät (ggf. unter mehreren medizinischen Geräten) betrifft, und der in der Datenbearbeitungseinheit entsprechend abgelegt werden muss. Unter einer bewirkten Aktion ist irgendein Vorgang seitens der Datenverarbeitungseinheit zu verstehen, der durch die Eingabe an dem zumindest einen medizinischen Gerät ausgelöst wird, beispielsweise das Abspeichern eines Parameters oder Geräte-Zustands, das Ändern eines Steuerungsparameters oder eines Steuerungsschritts und dergleichen.

In anderen Worten hat das medizinische (Behandlungs-)Gerät bevorzugt eine Ein- und ggf. Ausgabeeinheit oder eine (individuelle) Schnittstelle zu einem gerätebezogenen (mobilen) Ein- und ggf. Ausgabegerät (Tablet, Laptop, Mobiltelefon, etc.) für ein (manuelles) Ein- und ggf. Ausgeben von Informationen bzw. Daten in das medizinische (Behandlungs-)Gerät. Die Datenverarbeitungseinheit, bzw. der zentrale Server, bzw. zentraler Computer hat Hardware mit der Ein- und/oder Ausgabeeinheit zur Ein- und/oder Ausgabe von Informationen in die Datenverarbeitungseinheit selbst. Informationen, die in die Ein- und/oder Ausgabeeinheit der Datenverarbeitungseinheit eingebbar sind, können über die (systemeigene) Datenverbindung (unterschiedlich zu der Behandlungsgeräte-seitig ggf. vorhandenen individuellen Schnittstelle für ein mobiles Eingabegerät), die insbesondere eine kabellose Datenverbindung wie WIFI, Ethernet und/oder Bluetooth und/oder ein kabelbasiertes Bussystem wie USB sein kann, an das medizinische Gerät übertragen werden. Durch die Eingaben an der Datenverarbeitungseinheit selbst kann das medizinische Gerät also angesteuert / gesteuert werden. Beispielsweise können über die Datenverarbeitungseinheit (Software-)Updates an das medizinische (Behandlungs-) Gerät geschickt werden und das (Behandlungs-)Gerät, z.B. Pumpe kann somit (kabellos bzw. remote) über die Datenverarbeitungseinheit aktualisiert werden. Informationen, die in die Ein- und/oder Ausgabeeinheit des jeweiligen medizinischen Geräts eingegeben werden, können (in die entgegengesetzte Richtung) über die Datenverbindung von dem medizinischen Gerät an die Datenverarbeitungseinheit übertragen werden. Somit kann auch die Datenverarbeitungseinheit über diese Eingaben an dem medizinischen (Behandlungs-)Gerät angesteuert werden. Die Eingaben an dem medizinischen Gerät bzw. an der Bedieneinheit (Ein- und ggf. Ausgabegerät) des medizinischen Geräts werden von der Datenverarbeitungseinheit als Befehl verarbeitet. Insbesondere kann durch Informationseingabe an dem medizinischen Gerät ein Defekt an dem medizinischen Gerät, insbesondere der medizinischen Pumpe, gemeldet werden. Durch die DefektMeldung / Defekt-Information kann eine (beeinflussende) Aktion an der Datenverarbeitungseinheit getriggert / ausgelöst werden, die beispielsweise eine Instandsetzung in Gang setzt. Die (zentrale) Datenverarbeitungseinheit kann also durch die Informationseingabe an dem medizinischen Gerät gesteuert werden.

In nochmals anderen Worten ermöglichen die jeweiligen Ein- und/oder Ausgabegeräte / Bedieneinheiten (jeweils) an dem zumindest einen (dezentralen) medizinischen (Behandlungs-)Gerät und auch an der (zentralen) Datenverarbeitungseinheit sowie die Datenverbindung zwischen dem medizinischen Gerät und der Datenverarbeitungseinheit, also eine aktive bidirektionale (d.h. in beide Richtungen wirkende) Kommunikation zwischen dem medizinischen Gerät und der (zentralen) Datenverarbeitungseinheit als zentrale (digitale) Plattform. Die Datenverarbeitungseinheit ist also über die (integrale oder separate) Bedieneinheit des jeweiligen medizinischen (Behandlungs-)Geräts vergleichbar mit einer Fernsteuerung ansteuerbar und/oder mit einem aktiven Nachrichten- und Kommunikationsdienst.

Ein Kern der Offenbarung liegt somit darin, dass die Datenverarbeitungseinheit als die zentrale Plattform über das jeweilige medizinische (Behandlungs-)Gerät, wie z.B. eine Pumpe in der Funktion einer (Satelliten-) Workstation steuerbar ist, indem Eingaben, die am jeweiligen medizinischen (Behandlungsgerät) getätigt werden, an die zentrale Datenverarbeitungseinheit als Plattform übertragbar sind und über diese Eingaben die Datenverarbeitungseinheit betätigt wird.

Das medizinische Gerät kann insbesondere eine medizinische Pumpe, vorzugsweise eine Spritzen- oder Infusionspumpe sein. Das medizinische Gerät kann aber auch ein anderes medizinisches Instrument oder Gerät wie ein medizinischer Roboter, ein Endoskop, eine Überwachungsvorrichtung wie Blutdruck-Messgerät, Herzfrequenz-Messgerät, Beatmungsgerät oder eine extrakorporale Blutbehandlungsmaschine sein. Das medizinische Gerät kann wiederum ein Gerät sein, das in einem anderen/übergeordneten medizinischen Gerät verbaut ist. Beispielsweise kann das medizinischen Geräte eine Pumpe, ggf. Infusionspumpe, Spritzenpumpe, Förderpumpe, etc. sein, wohingegen das andere, übergeordnete medizinische Gerät beispielsweise eine extrakorporale Blutbehandlungsmaschine, Herz-Lungen-Maschine oder dergleichen Einrichtungen sein kann, in denen das medizinische Gerät verbaut/angeschlossen ist. Relevant ist lediglich, dass das medizinische Gerät eine (integrale oder mobile, Satellit-artige ) Bedieneinheit als Ein- und ggf. Ausgabegerät und eine (Daten-)Schnittstelle mit der Datenverarbeitungseinheit aufweisen kann bzw. mit einer solchen, dezentralen Bedieneinheit gekoppelt/koppelbar ist, wobei das medizinische Gerät bzw. dessen (Daten-) Schnittstelle dafür vorgesehen ist, an der/dessen Bedieneinheit eingegebene (Steuer-)Informationen auch in Richtung hin zur zentralen Datenverarbeitungseinheit zu übermitteln.

Dies hat gegenüber dem Stand der Technik den Vorteil, dass die Bedieneinheit des, vorzugsweise jedes oder ausgewählten (dezentralen) medizinischen Geräts dafür ausgebildet ist, um quasi für ein zumindest eingeschränktes oder vollumfängliches aktives Beeinflussen (Programmieren) der (zentralen) Datenverarbeitungseinheit vorzugsweise zusätzlich oder sogar alternativ zu dessen eigener Ein- und ggf. Ausgabeeinheit verwendet zu werden/verwendbar ist.

Die Bedieneinheit kann dabei insbesondere ein (Touch-)Bildschirm an dem medizinischen Gerät sein. Ferner kann das medizinische Gerät auch Eingabetasten oder -knöpfe aufweisen, durch die der Nutzer Eingaben in das medizinische Gerät tätigen kann. Wenn das medizinische Gerät eine medizinische Pumpe ist, dann kann der (Touch-)Bildschirm insbesondere in einer Frontklappe der Pumpe angeordnet sein. Die Eingabetasten können dabei neben dem Bildschirm angeordnet sein. Bei den Eingabetasten kann es sich auch um einen oder mehrere Bedienelemente handeln. Ferner kann die Bedieneinheit auch Leuchten aufweisen, über die insbesondere Signale ausgegeben oder angezeigt werden können.

Die Datenverarbeitungseinheit kann insbesondere ein Server oder ein Computer sein, der mit der Anzahl, vorzugsweise Mehrzahl von medizinischen Geräten, wie Pumpen (datenübertragbar) verbunden ist. Die Datenverarbeitungseinheit kann dabei insbesondere eine Datenverarbeitungseinheit-eigene (zentrale) Ein- und/oder Ausgabeeinheit aufweisen, um Ein- und Ausgaben an den Server bzw. Computer (direkt/unmittelbar) zu tätigen. Die Ein- und/oder Ausgabeeinheit kann dabei beispielsweise eine Kombination aus Maus, Tastatur und ein Bildschirm und/oder ein Touchscreen sein. Eine sprachgesteuerte Ein- und Ausgabe ist auch denkbar.

Die Datenverbindung (des Systems) kann dabei insbesondere eine WIFI und/oder eine Ethernet-Verbindung sein. Natürlich ist aber auch eine (drahtlose) Datenverbindung über Bluetooth, NFC und/oder eine kabelgebundene Datenverbindung über USB oder ein anderes Bussystem möglich.

Die individualisierten Informationen können beispielsweise Identifikationsinformationen des medizinischen Geräts, insbesondere der medizinischen Pumpe wie eine Seriennummer und/oder eine Modellnummer des medizinischen Geräts, insbesondere der Pumpe sein. Durch die Identifikationsinformationen kann die Datenverarbeitungseinheit ein gesuchtes medizinisches Gerät identifizieren, das mit der Datenverarbeitungseinheit verbunden ist. Die individualisierten Informationen können ferner Defekt-Informationen des medizinischen Geräts sein. Die Defekt-Informationen können eine Art des Defekts, die Seriennummer des defekten medizinischen Geräts, eine Reparaturanweisung und/oder eine Aufforderung sein, einen Nutzer zu alarmieren oder das Gerät zu sperren.

Ferner können die individualisierten Informationen auch durch einen Scan eines computerlesbaren Codes, beispielsweise eines QR-Codes oder eines Barcodes, erfassbar sein. Durch den Scan beispielsweise eines medizinischen Produkts oder des medizinischen Geräts kann das korrekte Produkt oder Gerät identifiziert werden. Anschließend kann das identifizierte Produkt oder Gerät (von der Datenverarbeitungseinheit) angesteuert werden.

Durch die bidirektionale Kommunikation kann die Datenverarbeitungseinheit durch das medizinische Gerät gesteuert werden. Beispielsweise kann der Defekt an dem medizinischen Gerät gemeldet werden. Die Defektmeldung kann an der Datenverarbeitungseinheit die Aktion auslösen, dass eine Reparatur oder Instandsetzung des medizinischen Geräts veranlasst wird. Ferner können durch die bidirektionale Kommunikation auch Informationen von der Datenverarbeitungseinheit an das medizinische Gerät gesendet werden. Beispielsweise kann ein bestimmtes Gerät in einer Mehrzahl an Geräten ausgewählt und erkennbar gemacht werden.

Die Aufgabe der vorliegenden Offenbarung wird ferner durch ein (medizinisches) Kennzeichnungsverfahren (im Sinne von Kenntlichmachung, Markierung) eines definierten / vorbestimmten / auszuwählenden / gesuchten medizinischen (Behandlungs-)Geräts aus einer Mehrzahl von medizinischen (Behandlungs-)Geräten gelöst, wobei die medizinischen Geräte jeweils über die (systemeigene) Datenverbindung mit der Datenverarbeitungseinheit verbunden sind. Das offenbarungsgemäße Kennzeichnungsverfahren weist die folgenden Schritte auf:
Gerätespezifische Informationen, insbesondere eine Geräte-ID und/oder eine Modell- und/oder Seriennummer eines medizinischen Geräts und/oder weitere Geräteinformationen, werden in die Ein- und/oder Ausgabeeinheit der Datenverarbeitungseinheit selbst eingegeben. Das gesuchte medizinische (Behandlungs-) Gerät wird anhand der eingegebenen (gerätespezifischen) Informationen von der Datenverarbeitungseinheit ausgewählt oder identifiziert. Für die Identifizierung des gesuchten medizinischen Geräts können die eingegebenen (Behandlungs-)gerätespezifischen Informationen insbesondere in einer Datenbank mit Informationen über die jeweiligen medizinischen Geräte verknüpft sein. Die (zentrale) Datenverarbeitungseinheit steuert das gesuchte medizinische Gerät (über die systemeigene Datenverbindung) an. Aufgrund der Ansteuerung des gesuchten medizinischen Geräts durch die Datenverarbeitungseinheit wird eine von dem Nutzer wahrnehmbare Kennzeichnung oder Markierung über eine Bedieneinheit, insbesondere über den (Touch-)Bildschirm des gesuchten medizinischen Geräts ausgegeben, wodurch das gesuchte medizinische Gerät in der Mehrzahl der medizinischen Geräte (von dem Nutzer) erkennbar / identifizierbar ist. Das gesuchte medizinische Gerät kann also durch die wahrnehmbare Kennzeichnung oder Markierung in Abgrenzung zu den anderen medizinischen Geräten gekennzeichnet und/oder markiert und identifizierbar gemacht werden.

Die Anzahl oder Mehrzahl von medizinischen Geräten ist also mit der (zentralen) Datenverarbeitungseinheit über die Datenverbindung verbunden. Dabei können die medizinischen Geräte insbesondere bei der Datenverarbeitungseinheit derart angemeldet sein, dass die einzelnen medizinischen Geräte durch die Datenverarbeitungseinheit identifizierbar und ansteuerbar sind. Dabei kann die Identifizierung der einzelnen medizinischen Geräte insbesondere über eine Zuordnung von Kabeln, die die medizinischen Geräte mit der Datenverarbeitungseinheit verbinden, oder durch ein elektronisches Identifizierungs- oder Zuordnungsverfahren erfolgen. Die Datenverarbeitungseinheit kann somit geeignet sein, die einzelnen verbundenen medizinischen Geräte einzeln anzusteuern.

Konkret kann ein Nutzer ein gewünschtes medizinisches Gerät an der Datenverarbeitungseinheit auswählen. Dazu kann der Nutzer beispielsweise einen Knopf auf der Ein- und/oder Ausgabeeinheit der Datenverarbeitungseinheit drücken oder eine Geräte-ID eingeben, die einem bestimmten medizinischen Gerät zugeordnet ist. Die Datenverarbeitungseinheit kann auf Basis der Eingabe des Nutzers das gesuchte medizinische Gerät ansteuern.

An dem angesteuerten medizinischen Gerät kann daraufhin die Markierung oder Kennzeichnung ausgegeben werden, die von dem Nutzer optisch, akustisch und/oder haptisch wahrnehmbar ist. Vorzugsweise kann die Bedieneinheit des medizinischen Geräts ein optisches Signal wie Blinken des Bildschirms und/oder ein akustisches Signal wie ein Piepsen oder Pfeifen ausgeben. Ein Vibrieren des medizinischen Geräts und/oder der Bedieneinheit kann ebenfalls möglich sein. Prinzipiell ist jedes Signal denkbar, das von dem Nutzer wahrnehmbar ist und das gesuchte Gerät von den anderen medizinischen Geräten abhebt oder unterscheidet.

Durch das Verfahren kann insbesondere das gesuchte medizinische Gerät einfach und schnell von dem Nutzer gefunden und identifiziert werden. Somit kann z.B. besonders ein defektes Gerät oder ein Gerät, das gewartet werden muss, schnell erkannt werden.

Ferner betrifft die vorliegende Offenbarung ein (medizinisches) (Defekt-)Meldeverfahren, um einen Defekt bei einem medizinischen Gerät zu melden / anzuzeigen. Defekt-Informationen über einen Defekt an dem medizinischen Gerät werden in die Bedieneinheit des medizinischen Geräts eingegeben, an dem der Defekt aufgetreten ist. Die eingegebenen Defekt-Informationen werden über eine Datenverbindung, die ggf. Teil des medizinischen Systems ist, an die Datenverarbeitungseinheit übermittelt. Die übermittelten Defekt-Informationen bewirken eine Aktion an der Datenverarbeitungseinheit. Die übermittelten Defekt-Informationen können ferner über eine Ein- und/oder Ausgabeeinheit der Datenverarbeitungseinheit (an den Nutzer) ausgegeben werden, um auf einen Defekt an dem medizinischen Gerät hinzuweisen.

Vorzugsweise kann die Aktion der Datenverarbeitungseinheit darin bestehen, auf Basis der übermittelten Defekt-Informationen eine Reparatur oder eine Instandsetzung des medizinischen Gerätes zu veranlassen. Dazu kann beispielsweise (automatisiert bzw. durch die Datenverarbeitungseinheit veranlasst) ein Techniker verständigt oder informiert werden, der das medizinische Gerät repariert.

Ferner kann die Aktion an der Datenverarbeitungseinheit auch darin bestehen, den gemeldeten Defekt in eine Datenbank der Datenverarbeitungseinheit einzutragen. Dadurch kann in der Datenverarbeitungseinheit gespeichert oder hinterlegt sein, dass das medizinische Gerät defekt ist. Somit kann die Datenverarbeitungseinheit dem Nutzer den Defekt anzeigen, sollte das betreffende medizinische Gerät zu einem späteren Zeitpunkt ausgewählt oder angesteuert werden. Ferner kann das defekte medizinische Gerät derart durch die Datenverarbeitungseinheit gesperrt werden, dass eine Benutzung unmöglich ist, bis eine Reparatur durchgeführt wurde. Zumindest kann bei einer Benutzung des defekten Geräts gewarnt werden, dass ein Defekt vorliegt.

Durch das (medizinische) Meldeverfahren kann der Nutzer beispielsweise den Defekt direkt an dem medizinischen Gerät eingeben und die Reparatur anzuweisen. Eine Eingabe des Defekts an der Datenverarbeitungseinheit kann dadurch überflüssig werden. Ein manuelles Anweisen der Reparatur oder Instandsetzung durch den Nutzer kann somit ebenfalls entfallen.

Vorteilhafte Weiterbildungen der vorliegenden Offenbarung sind Gegenstand der beigefügten Unteransprüche.

Nach einem optionalen Aspekt der vorliegenden Offenbarung können die individualisierten (oder auch standardisierte) Informationen, die in die Ein- und/oder Ausgabeeinheit der Datenverarbeitungseinheit eingebbar sind, Erkennungs-Informationen oder gerätespezifische Informationen seien, über die das gesuchte medizinische Gerät durch die Datenverarbeitungseinheit identifizierbar oder ansteuerbar ist. Durch die eingegebenen Informationen kann die Datenverarbeitungseinheit also das gesuchte medizinische Gerät identifizieren, ansteuern und in der Mehrzahl von medizinischen Geräten erkennbar machen.

In anderen Worten ausgedrückt können die gerätespezifischen Informationen, durch die das medizinische Gerät identifizierbar ist, (von dem Nutzer) in die Ein- und/oder Ausgabeeinheit der Datenverarbeitungseinheit eingegeben werden. Die eingegebenen Informationen können von der Datenverarbeitungseinheit verarbeitet werden. Die Datenverarbeitungseinheit kann auf Basis der gerätespezifischen Informationen das medizinische Gerät identifizieren, das von dem Nutzer gesucht wird und das gesuchte identifizierte medizinische Gerät ansteuern.

Vorzugsweise weist die Datenverarbeitungseinheit eine (hinterlegte) Datenbank auf, in der die gerätespezifischen Informationen, wie z.B. eine Geräte-ID einem bestimmten medizinischen Gerät zugeordnet ist. Die Datenverarbeitungseinheit kann dafür angepasst sein, anhand der eingegebenen Geräte-ID das bestimmte /gesuchte medizinische Gerät zu ermitteln und anzusteuern. Optional können die eingegebenen Erkennungs-Informationen über die Datenverbindung an das ermittelte medizinische Gerät übermittelt werden. Somit kann die Datenverarbeitungseinheit das gesuchte medizinische Gerät anhand der Geräte-ID ermitteln und ansteuern, um es in der Mehrzahl anderer medizinischer Geräte zu markieren.

Nach einem weiteren optionalen Aspekt der vorliegenden Offenbarung kann das medizinische Gerät derart durch die Datenverarbeitungseinheit angesteuert werden, dass über die Bedieneinheit (des ermittelten /gesuchten medizinischen Geräts) eine Kennzeichnung des gesuchten medizinischen Geräts ausgegeben wird. Vorzugsweise wird die Kennzeichnung über die Bedieneinheit ausgegeben, beispielsweise indem die Bedieneinheit aufblinkt oder grafische Informationen an der Bedieneinheit angezeigt werden. Dabei kann insbesondere ein (farbiger) Rand um die Bedieneinheit aufleuchten oder aufblinken. Dadurch kann ein Nutzer das gesuchte medizinische Gerät schnell und einfach identifizieren. Das kann besonders hilfreich sein, wenn ein einzelnes medizinisches Gerät in einer Menge / Mehrzahl von medizinischen Geräten identifiziert werden soll. Optional können die übermittelten Erkennungs-Informationen über die Bedieneinheit (des ermittelten /gesuchten medizinischen Geräts) anzeigbar sein. Ferner kann das gesuchte Gerät andere optische, akustische oder haptische Signale ausgeben.

Vorzugsweise können die individualisierten Informationen, die in die Bedieneinheit eingebbar sind, Gerätzustands-Informationen, insbesondere Defekt-Informationen, sein, die einen (aktuellen) Zustand des medizinischen Geräts anzeigen. Insbesondere können die Gerätzustands-Informationen anzeigen, dass das medizinische Gerät einen Defekt aufweist und/oder gewartet werden muss. Die Gerätzustands-Informationen können über die Datenverbindung an die Datenverarbeitungseinheit übermittelbar sein und eine Aktion an der Datenverarbeitungseinheit bewirken. Der Nutzer kann also beispielsweise direkt an dem medizinischen Gerät einstellen oder eingeben, dass ein Defekt vorliegt. Die Datenverarbeitungseinheit verarbeitet diese vom Nutzer eingegebene Information selbstständig. Die Aktion an der Datenverarbeitungseinheit kann beispielsweise das automatische Anweisen einer Reparatur des Geräts und/oder ein Sperren des Geräts sein.

Die Gerätzustands-Informationen können aber auch ein Hinweis auf eine anstehende (plan- oder turnusmäßige) Wartung oder eine Reinigung des medizinischen Geräts sein. In diesem Fall kann die Aktion der Datenverarbeitungseinheit drin bestehen, die Wartung oder Reinigung anzuweisen oder in einen Kalender einzutragen.

Vorzugsweise können die an die Datenverarbeitungseinheit übermittelten Gerätzustands-Informationen, an der Ein- und/oder Ausgabeeinheit der Datenverarbeitungseinheit anzeigbar sein. Die Gerätzustands-Informationen können insbesondere die Defekt-Informationen über die benötigte Wartung oder Reparatur des medizinischen Geräts sein.

Nach einem weiteren optionalen Aspekt der vorliegenden Offenbarung kann die Datenverarbeitungseinheit dafür angepasst sein, beim Empfang der Defekt-Informationen das medizinische Gerät auszuschalten, zu sperren und/oder eine Reparatur / Instandsetzung zu veranlassen. Die Datenverarbeitungseinheit kann also dafür sorgen, dass das medizinische Gerät in einem erkannten defekten Zustand nicht mehr benutzbar ist und veranlasst automatisch bzw. automatisiert, dass das defekte Gerät repariert wird.

Vorzugsweise kann die Bedieneinheit des medizinischen Geräts ein Touchscreen sein. Eingaben des Nutzers auf dem Touchscreen können dabei die Defekt-Informationen darstellen. Die Eingaben, insbesondere die Defekt-Informationen, können dabei die Aktion an der Datenverarbeitungseinheit bewirken. Wenn es sich bei den Eingaben um die Defekt-Informationen handelt, kann die Aktion insbesondere darin bestehen, die Reparatur des medizinischen Geräts zu veranlassen. Dadurch muss der Nutzer die Reparatur des medizinischen Geräts nicht mehr manuell veranlassen.

Vorzugsweise kann das System ein Trägersystem aufweisen, in das eine Mehrzahl von den medizinischen Geräten einsetzbar ist. Die Datenverarbeitungseinheit kann in dem Trägersystem angeordnet sein. Dadurch kann die Datenverarbeitungseinheit in einem bestehenden Bauteil angeordnet sein. Das System kann auf eine bestehende Verbindung der einzelnen Geräte zu dem Trägersystem zurückgreifen, beispielsweise für die Stromversorgung oder für eine Übertragung von Sensordaten.

Die Aufgaben der vorliegenden Offenbarung werden hinsichtlich des computerlesbaren Speichermediums und hinsichtlich des Computerprogramms dadurch gelöst, dass diese Befehle umfassen, die bei einer Ausführung durch einen Computer diesen veranlassen, die medizinischen Verfahren gemäß der vorliegenden Offenbarung auszuführen.

Jegliche Offenbarung im Zusammenhang mit dem medizinischen System gemäß der vorliegenden Offenbarung gilt ebenso für die medizinischen Verfahren gemäß der vorliegenden Offenbarung, insbesondere analog, wie auch umgekehrt.

### Kurzbeschreibung der Figuren

Die Offenbarung wird nachfolgend anhand eines bevorzugten Ausführungsbeispiels unter Bezugnahme auf die begleitenden Figuren näher erläutert.
Fig. 1 zeigt eine schematische Darstellung eines medizinischen Systems gemäß einer bevorzugten Ausführungsform der vorliegenden Offenbarung;
Fig. 2 zeigt eine perspektivische Darstellung einer medizinischen Pumpe als medizinisches Gerät gemäß der vorliegenden Offenbarung;
Fig. 3 zeigt eine Darstellung einer Bedieneinheit des medizinischen Geräts gemäß der vorliegenden Offenbarung;
Fig. 4 zeigt eine weitere Darstellung der Bedieneinheit des medizinischen Geräts gemäß der vorliegenden Offenbarung;
Fig. 5 zeigt eine perspektivische Darstellung eines medizinischen Systems gemäß einer Ausführungsform der vorliegenden Offenbarung;
Fig. 6 zeigt ein Ablaufdiagramm für ein medizinisches Kennzeichnungsverfahren gemäß einer bevorzugten Ausführungsform der vorliegenden Offenbarung; und
Fig. 7 zeigt ein Ablaufdiagramm für ein medizinisches Meldeverfahren gemäß einer bevorzugten Ausführungsform der vorliegenden Offenbarung.

### Detaillierte Beschreibung der Figuren

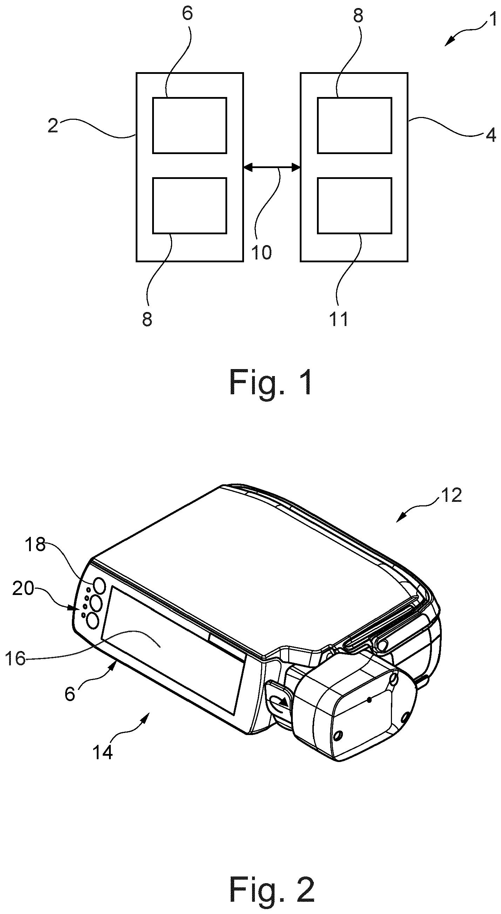

Fig. 1 zeigt ein medizinisches (Steuerung-/Überwachungs-/Kontroll-) System 1 mit zumindest einem (dezentralen, ggf. mobilen) medizinischen (Behandlungs-) Gerät 2 und einer (zentralen, ggf. stationären) Datenverarbeitungseinheit 4. Das medizinische Gerät 2 ist dabei vorzugsweise eine medizinische Spritzen- oder Infusionspumpe. Das medizinische Gerät 2 weist eine eigene Bedieneinheit 6 zur Ein- und ggf. Ausgabe von Informationen auf oder ist mit einer solchen, Geräte-eigenen (mobilen) Bedieneinheit 6 über eine Geräte-eigene Datenverbindung (Kabel, Bluetooth, etc.) gekoppelt. Die Datenverarbeitungseinheit 4 weist ebenfalls eine Ein- und ggf. Ausgabeeinheit 8 auf. Über die Ein- und ggf. Ausgabeeinheit 8 können Ein- und ggf. Ausgaben an die Datenverarbeitungseinheit 4 getätigt werden. Die Datenverarbeitungseinheit 4 und das medizinische Gerät 2 sind über eine Datenverbindung 10 vorzugsweise als weiterer Bestandteils des medizinischen Systems 1 miteinander derart verbunden, dass Daten und/oder Informationen von dem zumindest einen medizinischen Gerät 2 zur Datenverarbeitungseinheit 4 und umgekehrt und vorzugsweise von dem einen medizinischen Gerät 2 zu einem anderen medizinischen Gerät übertragen werden können.

Die Datenverarbeitungseinheit 4 kann vorzugsweise ein (zentraler) Server, ein Computer, eine digitale Plattform oder eine zentrale Steuereinheit sein. Die Datenverarbeitungseinheit 4 weist eine Speichereinheit auf oder ist mit einer solchen gekoppelt, in der eine Datenbank hinterlegt oder gespeichert ist.

Das medizinische Gerät 2 kann anstelle der vorstehend genannten Pumpen aber auch ein Operationsroboter, eine robotergestützte Visualisierungseinheit und/oder ein anderer medizinischer Roboter mit einer Bedieneinheit 6 sein.

Bei der Datenverbindung 10 kann es sich insbesondere um eine draht- oder kabellose Datenverbindung über WIFI, Bluetooth und/oder NFC und/oder eine kabelgebundene Datenverbindung über USB, Ethernet oder ein anderes Bussystem handeln.

Fig. 2 zeigt das medizinische Gerät 2 in einer beispielhaften Ausführungsform. Bei dieser Ausführungsform handelt es sich um eine medizinische Spritzenpumpe 12. Die Spritzenpumpe 12 weist in allgemein bekannter Weise eine Frontklappe 14 auf, durch die eine Spritze (nicht dargestellt) in der Spritzenpumpe fixiert werden kann. Die Frontklappe 14 weist ferner die Bedieneinheit 6 auf bzw. die Bedieneinheit 6 kann in die Frontklappe 14 integriert sein. Die Bedieneinheit 6 weist dabei einen Bildschirm 16, (Eingabe-)Tasten 18 und weitere Leuchten 20 auf. Der Bildschirm 16 kann als Touch-Bildschirm ausgeführt sein, sodass über den Bildschirm 16 Ein- und Ausgaben getätigt werden können. Über die Tasten 18 können Eingaben für die Spritzenpumpe 12 getätigt werden.

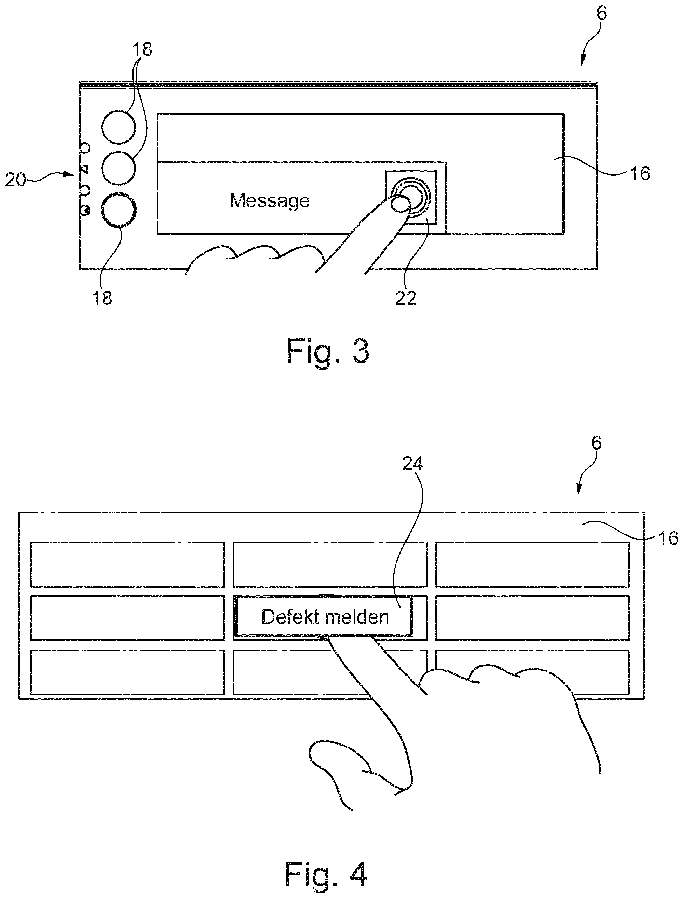

Fig. 3 zeigt die Bedieneinheit 6 im Detail. Die Bedieneinheit 6 hat den Bildschirm 16, die Eingabetasten 18 und die Leuchten 20. Auf dem Bildschirm 16 kann ein Dialogfenster 22 aufleuchten, dass einem Nutzer anzeigt, dass eine Meldung von der Datenverarbeitungseinheit 4 übermittelt wurde. Die Meldung der Datenverarbeitungseinheit 4 kann beispielsweise sein, dass das medizinische Gerät 2, welches bereits in der Datenverarbeitungseinheit 4 als ein Peripheriegerät angemeldet ist, (vom Nutzer) in der Datenverarbeitungseinheit 4 ausgewählt wurde. Wenn das medizinische Gerät 2 in der Datenverarbeitungseinheit 4 ausgewählt wurde, wird beispielsweise die Meldung beispielsweise auf dem Bildschirm 16 des medizinischen Geräts 2 ausgegeben, oder es wird ein anderweitiges Signal ausgelöst (Leuchte, Signalton, etc.), welches dieses eine medizinische Gerät 2 markiert, sodass der Nutzer das ausgewählte medizinische Gerät 2 identifizieren bzw. die Auswahl des richtigen Geräts am ausgewählten Gerät selbst verifizieren kann. Der Bediener muss sich also in diesem Fall nicht nur auf die Ausgabe an der zentralen Datenverarbeitungseinheit verlassen.

Fig. 4 zeigt ebenfalls den Bildschirm 16 der Bedieneinheit 6. Auf dem Bildschirm 16 ist ein weiteres Dialogfenster 24 angezeigt. Das Dialogfenster 24 hat beispielsweise den Text "Defekt melden". Wird das Dialogfenster 24 auf dem Touch-Bildschirm 16 von dem Nutzer aktiviert, dann wird diese Defekt-Information an die Datenverarbeitungseinheit 4 übermittelt. Die übermittelte Defekt-Information löst dann die Aktion an der Datenverarbeitungseinheit 4 aus. Die Aktion kann beispielsweise sein, dass die Datenverarbeitungseinheit 4 die Reparatur des medizinischen Geräts 2 anweist oder beauftragt.

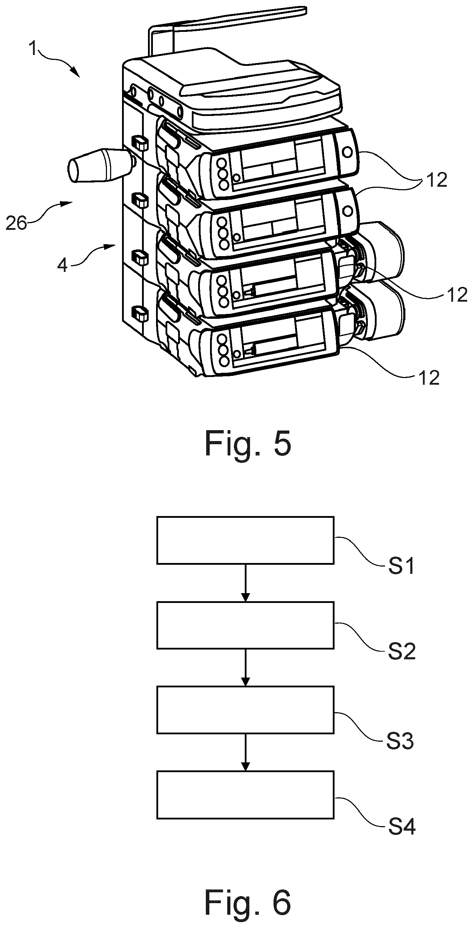

Fig. 5 zeigt eine Anordnung von mehreren medizinischen Geräten in Form von Pumpen 12 in einem gemeinsamen Trägersystem (Geräte-Rack) 26. Das Trägersystem 26 kann die Datenverarbeitungseinheit 4 aufweisen. Das hat den Vorteil, dass das Trägersystem 26 schon vorhanden ist und eine elektrische und datenübertragende Verbindung mit einer Anzahl an medizinischen Pumpen 12 aufweist. In diesem Fall ist also die zentrale Datenverarbeitungseinheit 4 sowie die Datenverbindung 10 in dem Trägersystem 26 verbaut/integriert oder daran montiert und damit zusammen mit dem Trägersystem 26 ggf. mobil, wohingegen die medizinischen Geräte 12 bezüglich des Trägersystems 26 grundsätzlich beweglich sind und nach Bedarf in das Trägersystem 26 eingesetzt und mit der Datenverbindung gekoppelt werden können.

Fig. 6 zeigt ein Ablaufdiagramm für ein (medizinisches) Kennzeichnungsverfahren (in Sinn der vorstehenden Definition) eines definierten / vorbestimmten / auszuwählenden / gesuchten medizinischen Geräts 2 aus einer Mehrzahl von medizinischen Geräten 2. Die einzelnen medizinischen Geräte 2 sind jeweils über die Datenverbindung 10 mit der Datenverarbeitungseinheit 4 verbunden (und bereits angemeldet), sodass Daten bzw. Informationen von der Datenverarbeitungseinheit zu den einzelnen medizinischen Geräten 2 übermittelt werden können.

In Schritt S1 gibt ein Nutzer hierzu gerätespezifische Informationen beispielsweise direkt in die Ein- und ggf. Ausgabeeinheit 8 der Datenverarbeitungseinheit 4 ein. Über die gerätespezifischen Informationen kann das bestimmte gesuchte medizinische Gerät 2 aus der Mehrzahl an medizinischen Geräten 2 identifiziert werden. Die gerätespezifischen Informationen können dabei beispielsweise die Seriennummer des gesuchten medizinischen Geräts 2 sein.

In Schritt S2 wählt die Datenverarbeitungseinheit 4 anhand der eingegebenen gerätespezifischen Informationen das gesuchte medizinische Gerät 2 aus der Mehrzahl an medizinischen Geräten 2 aus. D.h. das gesuchte medizinische Gerät 2 wird anhand der übermittelten gerätespezifischen Informationen, insbesondere der Seriennummer, von der Datenverarbeitungseinheit 4 identifiziert.

In Schritt S3 steuert die Datenverarbeitungseinheit 4 das gesuchte und inzwischen identifizierte/aufgefundene medizinische Gerät 2 über die Datenverbindung 10 an.

In Schritt S4 wird eine Kennzeichnung oder Markierung über die Bedieneinheit 6 des gesuchten/aufgefundenen medizinischen Geräts 2 ausgegeben, d.h. das gesuchte/aufgefundene medizinische Gerät 2 kann gekennzeichnet/markiert werden, beispielsweise in dem sich das ausgewählte/gesuchte medizinische Gerät durch ein Signal zu erkennen gibt. Die Kennzeichnung kann dabei insbesondere eine grafische Markierung wie ein Blinken an dem Bildschirm 16 der Bedieneinheit 6 des medizinischen Geräts 2 sein. So kann der Bildschirm 16 beispielsweise mit einem roten Rahmen umrandet sein, der (auf-)blinkt, wenn eines der medizinischen Geräte 2 als das gesuchte medizinische Gerät 2 identifiziert wurde. Ferner können die Leuchten 20 des medizinischen Geräts 2 aufleuchten oder aufblinken, um das medizinische Gerät 2 zu kennzeichnen.

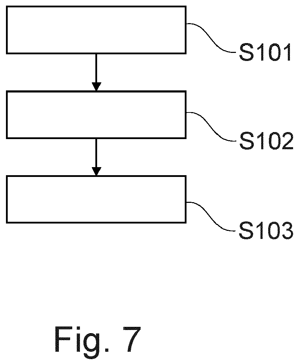

Fig. 7 zeigt ein (medizinisches) (Defekt-)Meldeverfahren im Falle eines Defekts bei dem medizinischen Gerät 2. Das Meldeverfahren weist die folgenden Schritte auf. In Schritt S100 gibt ein Nutzer (manuell) Defekt-Informationen in die Bedieneinheit 6 des medizinischen Geräts 2 ein, an dem ein Defekt aufgetreten ist. Dabei kann der Defekt des medizinischen Geräts 2 durch den Nutzer erkannt werden und/oder durch das Gerät 2 selbst angezeigt oder gemeldet werden, dass ein Defekt vorliegt. In Schritt S 101 werden die eingegebenen Defekt-Informationen über die Datenverbindung 10 von dem medizinischen Gerät 2 an die Datenverarbeitungseinheit 4 übermittelt. In Schritt S102 bewirken die übermittelten Defekt-Informationen eine Aktion an der Datenverarbeitungseinheit 4. Die übermittelten Defekt-Informationen können ferner durch die Ein- und/oder Ausgabeeinheit 8 der Datenverarbeitungseinheit 4 (an den Nutzer) ausgegeben, um auf den Defekt an dem medizinischen Gerät 2 hinzuweisen. Die Defekt-Informationen können insbesondere durch ein Dialogfenster an der Ein- und/oder Ausgabeeinheit 8 der Datenverarbeitungseinheit 4 angezeigt werden. Über die Defekt-Informationen kann beispielsweise die Seriennummer des defekten Geräts 2 ausgegeben werden, eine Information, welcher Fehler vorliegt und/oder ob eine Instandsetzung notwendig ist. Ferner kann über die Defekt-Information selbstständig technisches Personal mit der Reparatur oder Instandsetzung des medizinischen Geräts 2 beauftragt werden oder das defekte Gerät 2 für eine Benutzung gesperrt werden. Die Datenverarbeitungseinheit 4 kann die Information über den Defekt des medizinischen Geräts 2 in der Datenbank hinterlegen oder speichern.

### Bezugszeichenliste

- 1: System
- 2: medizinisches Gerät
- 4: Datenverarbeitungseinheit
- 6: Bedieneinheit
- 8: Ein- und/oder Ausgabeeinheit der Datenverarbeitungseinheit
- 10: Datenverbindung
- 11: Speichereinheit
- 12: Spritzenpumpe
- 14: Frontklappe
- 16: Bildschirm
- 18: Eingabetasten
- 20: Leuchten
- 22: erstes Dialogfenster
- 24: weiteres Dialogfenster
- 26: Trägersystem

- S1: Schritt 1
- S2: Schritt 2
- S3: Schritt 3
- S4: Schritt 4

- S101: Schritt 101
- S102: Schritt 102
- S103: Schritt 103

## Patentansprüche

1. Medizinisches System (1) mit zumindest einem medizinischen Gerät (2), insbesondere einer Spritzen- oder Infusionspumpe, welches eine Bedieneinheit (6) aufweist und einer Datenverarbeitungseinheit (4), insbesondere einem (Cloud-)Server, welches eine Ein- und vorzugsweise Ausgabeeinheit (8) aufweist, in die Geräte-individualisierte Informationen eingebbar sind, wobei das zumindest eine medizinische Gerät (2) anhand der Geräte-individualisierten Informationen von der Datenverarbeitungseinheit (4) über eine Datenverbindung (10) ansteuerbar ist und vorzugsweise eingegebene und übermittelte Informationen weiter vorzugsweise an der Bedieneinheit (6) anzeigbar sind, **dadurch gekennzeichnet, dass**
die Datenverarbeitungseinheit (4) über vorzugsweise Geräte-individualisierte Informationen, die in die Bedieneinheit (6) des zumindest einen medizinischen Geräts (2) eingebbar und über die Datenverbindung (10) an die Datenverarbeitungseinheit (4) übermittelbar sind, ansteuerbar ist und die übermittelten, vorzugsweise Geräte-individualisierten Informationen eine Aktion an der Datenverarbeitungseinheit (4) bewirken.

2. Medizinisches System (1) nach Anspruch 1, **dadurch gekennzeichnet, dass** die Geräte-individualisierten Informationen, die in die Ein- und vorzugsweise Ausgabeeinheit (8) der Datenverarbeitungseinheit (4) eingebbar sind, gerätespezifische Informationen, insbesondere eine Geräte-ID und/oder weitere Geräteinformationen, sind, über die ein gesuchtes medizinische Gerät (2) über die Datenverarbeitungseinheit (4) identifizierbar ist.

3. Medizinisches System (1) nach Anspruch 2, **dadurch gekennzeichnet, dass** die Datenverarbeitungseinheit (4) eine Datenbank aufweist, in der die gerätespezifischen Informationen, insbesondere die Geräte-ID, bestimmten medizinischen Geräten (2) zugeordnet sind, und die Datenverarbeitungseinheit (4) dafür angepasst ist, anhand der eingegebenen gerätespezifischen Informationen das gesuchte medizinische Gerät (2) zu ermitteln und die Erkennungs-Informationen an das ermittelte medizinische Gerät (2) zu übermitteln.

4. Medizinisches System (1) nach Anspruch 2 oder 3, **dadurch gekennzeichnet, dass** das identifizierte medizinische Gerät (2) derart von der Datenverarbeitungseinheit (4) ansteuerbar ist, dass das medizinische Gerät (2), insbesondere über die Bedieneinheit (6), eine Kennzeichnung ausgibt, vorzugsweise indem die Bedieneinheit (6) aufblinkt oder grafische Informationen an der Bedieneinheit (6) angezeigt werden, insbesondere ein (farbiger) Rand um die Bedieneinheit oder der Anzeige eines Pop-Up Feldes(6).

5. Medizinisches System (1) nach einem der Ansprüche 1 bis 4, **dadurch gekennzeichnet, dass** die individualisierten Informationen, die in die Bedieneinheit (6) eingebbar sind, Gerätzustands-Informationen, vorzugsweise Defekt-Informationen, sind, die einen aktuellen Zustand des medizinischen Geräts (2) abbilden, insbesondere, ob das das medizinische Gerät (2) einen Defekt aufweist und/oder gewartet werden muss, wobei die Gerätzustands-Informationen über die Datenverbindung (10) an die Datenverarbeitungseinheit (4) übermittelbar sind und die übermittelten Gerätzustands-Informationen eine Aktion an der Datenverarbeitungseinheit (4) bewirken.

6. Medizinisches System (1) nach Anspruch 5, **dadurch gekennzeichnet, dass** die an die Datenverarbeitungseinheit (4) übermittelten Gerätzustands-Informationen an der Ein- und/oder Ausgabeeinheit (8) der Datenverarbeitungseinheit (4) anzeigbar sind.

7. Medizinisches System (1) nach Anspruch 5 oder 6, **dadurch gekennzeichnet, dass** die Datenverarbeitungseinheit (4) dafür angepasst ist, beim Empfang der Gerätzustands-Informationen, insbesondere Defekt-Informationen, das medizinische Gerät (2) auszuschalten, zu sperren und/oder eine Reparatur / Instandsetzung zu veranlassen.

8. Medizinisches System (1) nach einem der Ansprüche 1 bis 7, **dadurch gekennzeichnet, dass** die Bedieneinheit (6) des medizinischen Geräts (2) ein Touchscreen (16) ist und Eingaben eines Nutzers auf dem Touchscreen die Aktion an der Datenverarbeitungseinheit (4) bewirken.

9. Medizinisches System (1) nach einem der Ansprüche 1 bis 8, **gekennzeichnet durch** ein Trägersystem (26), in das eine Mehrzahl von den medizinischen Geräten (2) einsetzbar ist und wobei das Trägersystem (26) die Datenverarbeitungseinheit (4) aufweist.

10. Medizinisches Kennzeichnungsverfahren eines gesuchten medizinischen Geräts (2) in einer Mehrzahl von medizinischen Geräten (2), die jeweils über eine Datenverbindung (10) mit einer Datenverarbeitungseinheit (4) verbunden sind, **gekennzeichnet durch** die folgenden Schritte:
- Eingabe von gerätespezifischen Informationen, insbesondere einer Modell-, einer Seriennummer und/oder einer Geräte-ID, in eine Ein- und/oder Ausgabeeinheit (8) der Datenverarbeitungseinheit (4);
- Auswahl eines gesuchten medizinischen Geräts (2) anhand der eingegebenen gerätespezifischen Informationen durch die Datenverarbeitungseinheit (4), insbesondere durch eine in der Datenverarbeitungseinheit (4) hinterlegte Datenbank, in der die eingegebenen gerätespezifischen Informationen mit Geräte-Informationen verknüpft sind;
- Ansteuern des ausgewählten medizinischen Geräts (2) durch die Datenverarbeitungseinheit (4) über die Datenverbindung (10);
- Ausgabe einer von einem Nutzer wahrnehmbaren Kennzeichnung durch eine Bedieneinheit (6) des gesuchten medizinischen Geräts (2), wodurch das gesuchte medizinische Gerät (2) in der Mehrzahl von medizinischen Geräten (2) erkennbar ist.

11. Medizinisches Kennzeichnungsverfahren nach Anspruch 10, wobei die von einem Nutzer wahrnehmbare Kennzeichnung ein optisches, akustisches und/oder haptisches Signal ist und insbesondere ein Blinken eines Bildschirms (16) der Bedieneinheit (6), ein Piepsen des gesuchten medizinischen Gerätes (2) und/oder eine Vibration des gesuchten medizinischen Gerätes (2) ist.

12. Medizinisches Meldeverfahren für ein medizinisches Gerät (2),
**gekennzeichnet durch** die folgenden Schritte:
- Eingabe von Defekt-Informationen in eine Bedieneinheit (6) eines medizinischen Geräts (2), an der ein Defekt aufgetreten ist;
- Übermittlung der eingegebenen Defekt-Informationen von dem medizinischen Gerät (2) an die Datenverarbeitungseinheit (4) über eine Datenverbindung (10);
- Auslösen einer Aktion an der Datenverarbeitungseinheit (4) aufgrund der übermittelten Defekt-Informationen, insbesondere Ausgabe der übermittelten Defekt-Informationen durch eine Ein- und/oder Ausgabeeinheit (8).

13. Medizinisches Meldeverfahren nach Anspruch 12, wobei die Aktion an der Datenverarbeitungseinheit (4) eine Reparatur des medizinischen Geräts (2) veranlasst, wenn ein Defekt an dem medizinischen Gerät (2) festgestellt wurde, und/oder das medizinische Gerät (2) sperrt.

14. Computerlesbares Speichermedium, umfassend Befehle, die bei Ausführung durch einen Computer diesen veranlassen, die Schritte des Kennzeichnungsverfahrens nach einem der Ansprüche 10 oder 11 und/oder des Meldeverfahrens nach einem der Ansprüche 12 oder 13 auszuführen.

15. Computerprogramm, umfassend Befehle, die bei Ausführung durch einen Computer diesen veranlassen, die Schritte des Kennzeichnungsverfahrens nach einem der Ansprüche 10 oder 11 und/oder des Meldeverfahrens nach einem der Ansprüche 12 oder 13 auszuführen.
